# EUROPÄISCHE PATENTSCHRIFT

(11) **EP 1 753 484 B1**
(45) Veröffentlichungstag und Bekanntmachung des Hinweises auf die Patenterteilung: **14.03.2012**
(21) Anmeldenummer: 05744790.6
(22) Anmeldetag: 14.05.2005
(51) Int. Cl.: A61M 1/16, A61M 1/36

(54) **HÄMODIALYSEGERÄT MIT NOTFALLAKTIVIERUNGSMITTEL**
HEMODIALYSIS UNIT COMPRISING EMERGENCY ACTIVATION MEANS
APPAREIL D'HEMODIALYSE COMPORTANT UN MOYEN D'ACTIVATION D'URGENCE

(30) Priorität: 27.05.2004 DE 102004026561
(43) Veröffentlichungstag der Anmeldung: 21.02.2007
(73) Patentinhaber: Fresenius Medical Care Deutschland GmbH, 61352 Bad Homburg v.d.H. (DE)
(72) Erfinder: DANIEL, Pia, 78351 Bodman (DE); MÜLLER, Carsten, 97502 Euerbach (DE)
(74) Vertreter: Ziermann, Oliver
(86) Internationale Anmeldenummer: PCT/EP2005/005322
(87) Internationale Veröffentlichungsnummer: WO 2005/118024

(56) Entgegenhaltungen:
- WO-A-96/41292
- US-A- 5 370 123
- US-A1- 2001 007 058

## Beschreibung

Die Erfindung betrifft ein Hämodialysegerät nach dem Oberbegriff des Anspruchs 1.

In der Hämodialyse wird Blut in einem extrakorporalen Kreislauf kontinuierlich einem Patient entnommen, durch einen Hämodialysator geleitet und dem Patienten wieder reinfundiert. Dabei wird ein Stoffaustausch durchgeführt, der dem der Nieren ähnlich ist. Der Hämodialysator besteht aus zwei durch eine semipermeable Membran getrennte Kammern, von denen die eine vom Blut und die andere von einer Reinigungsflüssigkeit - der Dialysierflüssigkeit - durchflossen wird. Die handelsüblichen Hämodialysatoren weisen hierfür meist Tausende von Hohlfasern auf, deren Wände semipermeabel sind. Das Blut wird durch den Innenraum der Hohlfasern geleitet, während die Dialysierflüssigkeit in meist gegenläufiger Richtung in den Faserzwischenraum eingespeist und abgeführt wird.

Die Dialysierflüssigkeit weist Konzentrationen von Blutinhaltsstoffen wie Elektrolyten auf, die in etwa denen eines Gesunden entsprechen, damit die entsprechenden Konzentrationen im Blut auf einem normalen Niveau gehalten werden können. Aus dem Blut zu entfernende Stoffe wie zum Beispiel Kreatinin oder Harnstoff sind in der Dialysierflüssigkeit nicht enthalten, wodurch diese allein wegen des Konzentrationsgradienten an der Membran durch Diffusion aus dem Blut entfernt werden. Mit Hilfe eines Druckgradienten wird dem Blut überschüssiges Wasser durch Konvektion beziehungsweise Ultrafiltration entzogen.

Zur Steuerung derartiger Vorgänge werden Hämodialysegeräte eingesetzt, die zumeist auch die Zubereitung der Dialysierflüssigkeit aus Wasser und Konzentraten mit der richtigen Zusammensetzung und Temperatur sicherstellen. Gleichzeitig sind diese Geräte zunehmend in der Lage, unterschiedlichste Überwachungsaktionen der Hämodialysebehandlung zu übernehmen, um eine Gefährdung für den Patienten so klein wie möglich zu halten und rechtzeitige Gegenmaßnahmen bei Auftreten von Komplikationen zu ermöglichen.

Unter den Komplikationen kommt den Blutdruckabfällen eine besondere Bedeutung zu. So treten bei 40-50% der Behandlungen Probleme dieser Art auf. In ca. 20-30% der Behandlungen muss die Dialyse durch den Anwender unterbrochen und der Blutdruckstatus des Patienten wieder hergestellt werden. Dabei muss der Anwender einen immer ähnlichen Ablauf an Aktionen an der Dialysemaschine durchführen, die ihn für ca. 1-2 Minuten allein mit der Bedienung der Dialysemaschine bindet, anstelle sich direkt den Patienten zuwenden zu können.

Diese Maßnahmen umfassen eine Reduktion des Blutflusses, um arterielle Alarme an den Blutdrucksensoren in der Blutzuführleitung des extrakorporalen Kreislaufes zu verhindern, ein Stoppen der Ultrafiltration, um einen weiteren Volumenentzug zu verhindern, ein Starten einer Blutdruckmessung, um den aktuellen Blutdruckstatus des Patienten zu gewinnen sowie die Gabe einer Substanz in das Blut des Patienten, um den Blutvolumenstatus des Patienten zu erhöhen. Dies kann durch Erhöhung der Konzentration eines Stoffes wie Kochsalz in der Dialysierflüssigkeit geschehen, wobei das Kochsalz ohne nennenswerte Volumenverschiebung allein durch den Konzentrationsunterschied durch die Membran ins Blut gelangt. Es kann aber auch durch eine Volumeninfusion in Form eines Bolus direkt in die Leitungen des extrakorporalen Blutkreislaufes oder durch die Membran des Dialysators geschehen.
In der EP 0 311 709 A1 wird ein Hämodialysegerät vorgestellt, bei dem der Blutdruck und der Herzschlag eines Patienten kontinuierlich überwacht werden. Falls die Messwerte außerhalb bestimmter Wertebereiche liegen, initiiert die Maschine selbstständig ohne die Einwirkung des Personals Gegenmaßnahmen, um den Kreislauf des Patienten zu stabilisieren. Diese Maßnahmen umfassen eine Verringerung der Ultrafiltration und eine Anhebung der Natriumkonzentration in der Dialyserflüssigkeit. Nach der Lehre der EP 0 311 709 A1 soll das Dialysepersonal erst nach erfolglosen, automatisch eingeleiteten Gegenmaßnahmen einbezogen werden.

Auch die EP 0 911 044 A1 der Patentanmelderin hat einen automatisierten Eingriff in die Behandlung zum Gegenstand. Mit Hilfe von Pulswellenlaufzeitmessungen kann der Blutdruck besonders gut kontinuierlich überwacht werden. Der Benutzer kann dabei vorgeben, welche Maßnahmen durch das Hämodialysegerät eingeleitet werden sollen, wenn es zu einem Blutdruckabfall kommt. Diese Maßnahmen umfassen eine Absenkung der Temperatur der Dialysierflüssigkeit, eine Abschaltung der Ultrafiltration, die Applikation eines Bolus von Kochsalzlösung und die Veränderung der Zusammensetzung der Dialysierflüssigkeit.

Um hypotonische Phasen zu vermeiden, schlägt die WO 94/27658 A1 eine manuell betätigbare Einrichtung bei einem Hämodialysegerät vor, bei deren Betätigung zusätzliches Natrium in die Dialyserflüssigkeit gegeben wird.

WO 96/41292 beschreibt ein Informations- Eingabe- System für eine Dialysemaschine, das für eine sichere Eingabe von Informationen sorgt. Die Eingabe kann dabei über einen Touchscreen erfolgen. Der Touchscreen zeigt verschiedene Symbole an, um dem Benutzer verschiedene Eingabemöglichkeiten anzubieten. Eine der Eingabemöglicbkeiten ist die Abfrage des Blutdrucks des Patienten, wenn diese Funktionalität vorgesehen ist und wenn eine Blutdruckmanschette an den Patienten angeschlossen ist.

US2001/0007058 beschreibt eine Dialysemaschine mit einer Fernsteuerungseinheit mit einer Einrichtung zur Beendigung der Dialysebehandlung die eine Zugabeeinheit für eine Salzlösung enthält Am Ende einer Blutbehandlung kann so Salzlösung von der Zugabeeinheit in den arteriellen Zweig eingeleitet werden und anschließend der extrakorporale Blutkreislauf von Blut entleert werden, während die Salzlösung das Blut verdrängt. Die Fernsteuerungseinheit dient der Automatisierung dieses Vorgangs, dazu steuert sie die Blutpumpe im extrakorporalen Blutkreislauf, Klemmen im arteriellen Zweig des extrakorporalen Blutkreislaufs sowie in der Leitung für Salzlösung in der Zugabeeinheit an. Es wird erwähnt, dass die Zugabe von Salzlösung auch in anderen Stadien der Behandlung erfolgen kann, etwa um einen Zustand der Dehydrierung zu behandeln.

Bei manuellen Eingriffen, denen in der Praxis nach wie vor große Bedeutung zukommt, müssen wie bereits ausgeführt oft mehrere Schritte nacheinander veranlasst werden, was eine gewisse Zeitspanne in Anspruch nimmt. Dabei bestätigt das Hämodialysegerät die einzelnen Maßnahmen unterschiedlich, worunter eine übersichtliche Informationsdarstellung und Benutzerführung leidet.

Der Erfindung liegt die Aufgabe zugrunde, ein gattungsgemäßes medizinisches Gerät derart weiter zu bilden, dass dem Benutzer eine schnelle und einfache Bedienung des Hämodialysegerätes zum Entgegenwirken von Blutdruckabfällen während einer Hämodialysebehandlung ermöglicht, wobei der Benutzer gleichzeitig eine übersichtliche Bestätigung der eingeleiteten Maßnahmen erhält.

Nach der Lehre der Erfindung wird diese Aufgabe durch ein Hämodialysegerät mit den Merkmalen des Anspruches 1 gelöst. Vorteilhafte Ausführungsformen der Erfindung sind Gegenstand der Unteransprüche.

Die Erfindung sieht bei einem Hämodialysegerät mit einem extrakorporalen Kreislauf, in dem Blut von einem Patienten zu der Blutkammer eines durch eine semipermeable Membran in zwei Kammern geteilten Hämodialysators über eine Blutzuführleitung und von der Blutkammer zu dem Patienten über eine Blutrückführleitung geführt wird und bei dem mit einer Blutpumpe das Blut durch den extrakorporalen Blutkreislauf gefördert wird, mit einer Zugabeeinheit zur Zugabe einer Substanz in das Blut des Patienten, mit einer Ultrafiltrationseinheit zum Entziehen von Flüssigkeit aus der zweiten Kammer des Dialysators, mit einer Blutdruckmesseinheit zum Messen des Blutdrucks des Patienten, mit einer Steuer- und Auswerteeinheit, die mit der Blutpumpe, der Zugabeeinheit, der Ultrafiltrationseinheit und der Blutdruckmesseinheit verbunden ist, mit einer Ausgabeeinheit zur Ausgabe von Information, die mit der Steuer- und Auswerteeinheit verbunden ist und mit einer Eingabeeinheit zur Eingabe von Daten und Anweisungen, die mit der Steuer- und Auswerteeinheit verbunden ist, vor, dass die Eingabeeinheit ein Notfallaktivierungsmittel umfasst, dass bei Betätigung ein Signal an die Steuer- und Auswerteeinheit abgibt, worauf diese von der Blutdruckmesseinheit einen Blutdruckmesswert anfordert, die Zugabeeinheit anweist, in vorbestimmter Art die Substanz in das Blut abzugeben sowie die Ausgabeeinheit anweist, in einem sich von dem während der Behandlung gezeigten Erscheinungsbild abweichenden Erscheinungsbild eine Statusinformation über die Zugabe der Substanz in das Blut und die Blutdruckmessung anzuzeigen.

Damit ermöglicht es die Erfindung dem Bedienpersonal beim Auftreten von Begleiterscheinungen, die auf eine hypotensive Episode hindeuten, durch Betätigung des bereitgestellten Notfallaktivierungsmittels mit einer einzigen Anweisung an das Hämodialysegerät die unmittelbar notwendigen Maßnahmen einzuleiten. Gleichzeitig wird dem Bedienpersonal eine automatische und eindeutige Bestätigung der eingeleiteten Maßnahmen ermöglicht, in dem die Ausgabeeinheit zum Beispiel auf dem Bildschirm des Hämodialysegerätes in einem sich von dem während der Behandlung gezeigten Erscheinungsbild abweichenden Erscheinungsbild eine Statusinformation über die eingeleiteten Schritte bereitstellt.

Als primär notwendige erste Maßnahmen sieht die Erfindung die Initiierung einer Blutdruckmessung und die Gabe einer Substanz an den Patienten zum Zwecke der Kreislaufstabilisierung vor. Bei der Blutdruckmessung kann die weit verbreitet Messung mit Manschette verwendet werden, die über eine Steuerung mit Luft unter Druck gesetzt und auch wieder entlastet werden kann. Entsprechende Sensoren können dann die Pulssignale aufnehmen. Dem Fachmann sind hierfür vielfältigste Mittel geläufig. Derartige Manschetten werden Dialysepatienten meist routinemäßig um den Oberam gebunden, um jederzeit eine Blutdruckmessung durchführen zu können. Es können aber auch andere Messmethoden zur Blutdruckmessung zum Einsatz kommen.

Die Zeitverzögerung einer Manschettenmessung spielt nach dem Konzept der Erfindung eine untergeordnete Rolle, da die einzelnen Schritte nach der Betätigung des Notfallaktivierungsmittel automatisch durchlaufen werden und die Gewinnung des Blutdruckmesswertes auch mit der Manschette nur wenige Sekunden in Anspruch nimmt.

Bei der Gabe der kreislaufstabilisierenden Substanz wird hauptsächlich zwischen zwei Methoden zu wählen sein. Entweder wird über die Steuer- und Auswerteeinheit des Hämodialysegerätes die Konzentration der Dialysierflüssigkeit verändert, wobei insbesondere eine Erhöhung der Natriumkonzentration in Betracht kommt. Oder es wird in das Blut des Patienten eine Infusion verabreicht, wofür sich wiederum eine entsprechende Kochsalzlösung anbietet. Bei der Infusion kann auf eine getrennte Infusionseinheit zurückgegriffen werden, die mit der Steuer- und Auswerteeinheit des Hämodialysegerätes verbunden ist. Bei heutigen Hämodialysegeräten ist es jedoch immer mehr verbreitet, dass diese Infusionslösung direkt vom Hämodialysegerät zubereitet werden kann, indem die im Gerät zubereitete Dialyserflüssigkeit dafür verwendet wird.

Herkömliche Hämodialysegräte stellen Messwerte und Behandlungsparameter wie Pumpenflüsse und Behandlungzeit aktuell bereit, wobei die Aktualisierung je nach Parameter verschiedenen Zeitzyklen unterliegt. Da sich die einzelnen Werte ohne weitere Kennzeichnung aktualisieren können, besteht die Gefahr, dass ein Benutzer bei Auslösung automatischer Vorgänge nicht unmittelbar erkennt, in welchem Status sich das Gerät befindet. Daher sieht die Erfindung zusätzlich vor, dass die Ausgabeeinheit des Hämodialysegeräts die Anzeige von Statusinformationen über die eingeleiteten Massnahmen in einem Erscheinungsbild veranlasst, die vom üblichen während der Behandlung gezeigten Erscheinungsbild abweicht. Die Ausgabe erfolgt dabei vorzugsweise auf einem Bildschirm, der ein mittlerweile weit verbreiteter Bestandteil von Dialysegeräten ist.

Bei der Anzeige des Blutdruckmesswertes bedeutet dies zum Beispiel, dass der zuletzt angezeigte Wert nicht einfach nur aktualisiert wird, sondern dass der Benutzer genau erkennen kann, dass es sich bei dem angezeigten Messwert um den Messwert handelt, dessen Bestimmung durch Betätigung des Notfallaktivierungsmittels ausgelöst wurde. Dies geschieht in einer vorteilhaften Ausführungsform durch die Bereitstellung eines neuen Ausgabefensters auf dem Bildschirm. Die angezeigte Statusinformation kann auch die qualitative Bestätigung der eingeleiteten Maßnahmen umfassen, wie zum Beispiel "Blutdruckmessung initiiert" oder "Bolusgabe initiiert".

In einer besonders vorteilhaften Ausführungsform ist der Bildschirm als berührungsempfindlicher Bildschirm (Touchscreen) auch als Teil der Eingabeeinheit ausgestaltet. Das Notfallaktivierungsmittel kann dann als ein Bereich auf dem Bildschirm mit einem entsprechenden Symbol markiert sein. Durch Berührung dieses Symbols sendet die Eingabeeinheit das Signal an die Steuer- und Auswerteeinheit ab, damit diese die entsprehenden Maßnahmen einleiten kann. Die sich darauf einstellende abweichende Erscheinungsform der Statusinformation kann neue Eingabebereiche vorsehen, die dem Bedienpersonal weitere Eingaben ermöglichen.

Schließlich können zusätzliche Maßnahmen durch die Betätigung des Notfallaktivierungsmittels in Gang gesetzt werden, wie zum Beispiel eine Reduzierung oder Abschaltung der Ultrafiltration oder der Blutförderung.

Im bisherigen Verlauf der Beschreibung der Erfindung ist auf die Bedeutung der Erfindung für die Hämodialyse hingewiesen worden. Bei der Hämodialyse wird streng genommen immer Dialysierflüssigkeit in die entsprechende Kammer des Hämodialysators eingeführt. Neben der Hämodialyse hat aber auch die Hämofiltration und die kombinierte Anwendung beider dieser Verfahren, die Hämodiafiltration, Bedeutung als Blutreinigungsverfahren. Bei der Hämofiltration wird der zweiten Kammer des Hämodialysators - nun streng genommen Hämofilter genannt - keine Dialysierflüssigkeit zugeführt, sondern nur Ultrafiltrat entzogen. Die Ultrafiltrationsrate liegt dabei weit über dem Wert, der sich aufgrund der Entfernung des Flüssigkeitsüberschusses im Blut des Patienten ergeben würde. Auf diese Weise werden mit dem Ultrafiltrat auch zu entfernende Substanzen in nennenswertem Umfang aus dem Blut entfernt. Gleichzeitig muss jedoch der Flüssigkeitshaushalt des Patienten ausgeglichen werden, was durch Zugabe einer entsprechenden Menge von physiologischer Substitutionslösung geschieht.

Bei der Hämofiltrationsbehandlung treten daher ähnliche Kreislaufbelastungen auf wie bei der Hämodialyse. Das Konzept der Erfindung lässt sich daher in naheliegender Weise auf ein Hämofiltrationsgerät übertragen. Wenn im Rahmen der Erläuterung der Erfindung im Folgenden einfacherweise von Hämodialyse gesprochen wird, soll damit die Hämofiltration beziehungsweise Hämodiafiltration in gleicher Weise mit einbezogen sein. Auch wird im Folgenden nicht zwischen einem Hämodialysator und einem Hämofilter unterschieden werden. Vielmehr wird allgemein der Begriff Hämodialysator verwendet.

Weitere Einzelheiten und Vorteile der Erfindung werden anhand eines in den Zeichnungen dargestellten Ausführungsbeispiels näher beschrieben. Es zeigen:
Fig. 1 eine Ausführungsform des erfindungsgemäßen Hämodialysegerätes in schematischer Darstellung,
Fig. 2 eine erste auf dem Bildschirm des in Fig. 1 dargestellten Hämodialysegerätes gezeigte Ansicht mit dem während einer Behandlung dargestellten Erscheinungsbild,
Fig. 3 eine zweite auf dem Bildschirm gezeigte Ansicht mit einem Erscheinungsbild, das sich nach der Betätigung des Notfallaktivierungsmittels einstellt und
Fig. 4 eine dritte auf dem Bildschirm gezeigte Ansicht, die zeitverzögert der zweiten Ansicht nachfolgt.

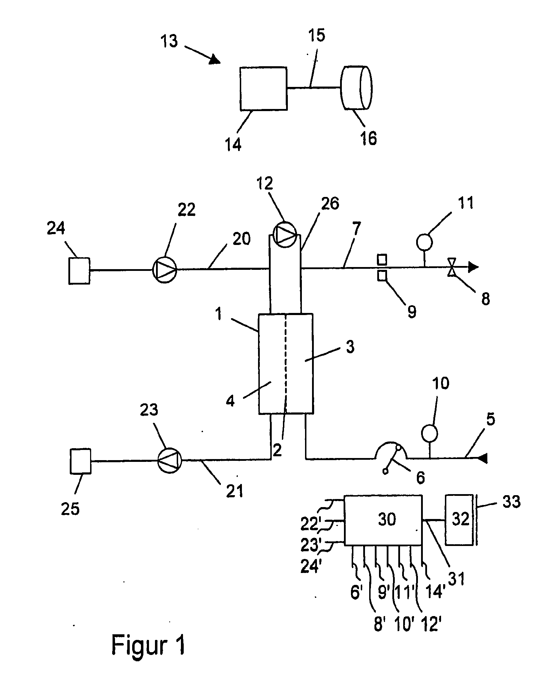

Anhand von Fig. 1 wird zunächst der prinzipielle Aufbau des erfindungsgemäßen Hämodialysegeräts erläutert. Bei der Hämodialyse wird Blut in einem extrakorporalen Kreislauf über eine Blutzuführleitung 5 einem Hämodialysator 1 zugeführt. Im Hämodialysator 1 trennt eine meist in Form vieler Hohlfasern ausgeführte semipermeable Membran 2 eine erste Kammer 3 (Blutkammer), die Teil des extrakorporalen Blutkreislaufs ist, von einer zweiten Kammer 4 (Dialysierflüssigkeitskammer), die Teil eines Dialysierflüssigkeitkreislaufes ist. Durch die semipermable Membran 2 treten aus dem Blut zu entfernende Stoffe in die Dialysierflüssigkeit über, die durch diese abgeführt werden. Gleichzeitig kann über einen Druckgradienten eine überschüssige Flüssigkeitsmenge aus dem Blut ultrafiltriert und über die abfließende Dialysierflüssigkeit entfernt werden. Schließlich kann auch ein umgekehrter Diffusionsgradient zum Beispiel für Natriumionen dafür verwendet werden, um diese Substanzen von der Dialysierflüssigkeit ins Blut zu überführen.

In der Blutzuführleitung 5 wird Blut durch eine als Rollenpumpe ausgestaltete Blutpumpe 6 gefördert. Das Blut verlässt die erste Kammer 3 des Hämodialysators 1 über die Blutrückführleitung 7, um wieder zum Patienten zurück geführt zu werden. An der Blutrückführleitung 7 ist eine venöse Absperrklemme 8 vorgesehen, mit der die Rückführung des Bluts insbesondere in Notfällen unterbrochen werden kann. Derartige Notfälle können z.B. auftreten, wenn durch einen Luftdetektor 9 Luft in der Blutrückführleitung 7 detektiert wird. Der Luftdetektor 9 umfasst auch Mittel, um die Präsenz von Blut in der Blutrückführleitung 7 zu erkennen.

An der Blutzuführleitung 5 ist ein arterieller Drucksensor 10 und an der Blutrückführleitung 7 ein venöser Drucksensor 11 vorgesehen.

Die zweite Kammer 4 des Hämodialysators wird von Dialysierflüssigkeit durchströmt, die über eine Dialysierflüssigkeitszuführleitung 20 von einer Dialysierflüssigkeitszubereitungseinheit 24 zugeführt und über eine Dialysierflüssigkeitsabführleitung 21 zu einem Abfluss 25 abgeführt wird. Die Dialysierflüssigkeit wird durch Förder- und Bilanziereinrichtungen 22 und 23 umgewälzt, wobei ein eventuell zu entfernendes Ultrafiltrat in der Menge genau erfasst werden kann. Dem Fachmann stehen zur Realiserung der Förder- und Bilanziereinrichtungen 22 und 23 verschiedenste Ausgestaltungen zur Verfügung, so dass an dieser Stelle von näheren Ausführungen abgesehen wird. Gleiches gilt auch für die Bereitstellung von Dialysierflüssigkeit durch die Dialysierflüssigkeitszubereitungseinheit 24. Beispielhaft wird an dieser Stelle auf ein Bilanzkammersystem hingewiesen, wie es in der US 4,267,040 beschrieben ist.

Auch zum Einsatz von Aktoren und Sensoren in einem Hämodialysegerät im Allgemeinen stehen dem Fachmann zahlreiche Möglichkeiten zur Verfügung, ohne dass hier im Detail darauf eingegangen werden muss. Die Darstellung in Fig. 1 ist auf einige wenige dieser Elemente beschränkt, die für die Erläuterung der Erfindung ausreichend sind.

Das Hämodialysegerät wird durch eine Steuer- und Auswerteeinheit 30 gesteuert und überwacht. Hierzu ist die Steuer- Auswerteeinheit 30 mit den einzelnen Aktoren und Sensoren des Gerätes mit Signalleitungen verbunden. Für die in Fig. 1 dargestellten Aktoren und Sensoren ist dies durch Bezugsziffern angedeutet, die neben der Bezugsziffer des betreffenden Aktors oder Sensors ein Apostroph aufweisen und die der Übersichtlichkeit halber nur an der Steuer- und Auswerteeinheit 30 eingezeichnet sind.

Die Steuer- und Auswerteeinheit 30 ist mit einer Ausgabe- und Eingabeeinheit 32 über eine Datenleitung 31 verbunden. Die Ausgabe- und Eingabeeinheit 32 umfasst einen als Touchscreen ausgebildeten Bildschirm 33. Auf dem Touchscreen werden von der Steuer- und Auswerteeinheit 30 gemeldete Informationen angezeigt, gleichzeitig werden über den Touchscreen von einer Bedienperson eingegebene Daten an die Steuer- und Auswerteeinheit 30 weitergeleitet.

Von der Dialysierflüssigkeitszuführleitung 20 zweigt eine Substituatleitung 26 ab, die in die Blutrückführleitung 7 mündet. In die Substituatleitung 26 ist eine Substituatpumpe 12 zur Förderung von Dialysierflüssigkeit als Substituat in den extrakorporalen Kreislauf des Hämodialysegerätes vorgesehen, die von der Steuer- und Auswerteeinheit 30 gesteuert wird.

Das in Fig. 1 gezeigte Hämodialysegerät kann bei Deaktivierung der Substituatpumpe 12 zur reinen Hämodialyse verwendet werden. Bei Unterbechung der Dialysierflüssigkeitsleitung 20 zwischen der Abzweigung der Substituatleitung 26 und der zweiten Kammer 4 durch ein nicht gezeigtes Ventil kann eine Hämofiltrationsbehandlung und bei gleichzeitiger Hämodialyse und Hämofiltration eine Hämodiafiltrationsbehandlung durchgeführt werden. In den Dialysierflüssigkeitszuführleitung 20 und/oder der Substituatleitung 26 können nicht näher gezeigte Filter zur Sterilfiltrierung der Dialysierflüssigkeit vorgesehen sein, wofür dem Fachmann mannigfaltige Lösungen zur Verfügung stehen.

An die Steuer- und Auswerteeinheit 30 ist eine Blutdruckmesseinheit 13 angeschlossen. Die Blutdruckmesseinheit 13 besteht aus einer Steuereinheit 14 und einer aufblasbaren Manschette 16, die an einem Arm des Patienten befestigt werden kann sowie mit einer Verbindungsleitung 15 mit der Steuereinheit 14 verbunden ist. Die Verbindungsleitung 15 umfasst die zur Messung notwendigen Luft- und Signalleitungen, damit die Steuereinheit 14 zusammen mit einem nicht gezeigten Kompressor eine Blutdruckmessung nach der Riva-Rocci-Methode durchführen kann. Wenn die Steuer- und Auswerteeinheit 30 eine Messung initiiert, wird der durch die Steuereinheit 14 bestimmte Blutdruckmesswert an die Steuer- und Messwerteinheit 30 zurückgemeldet.

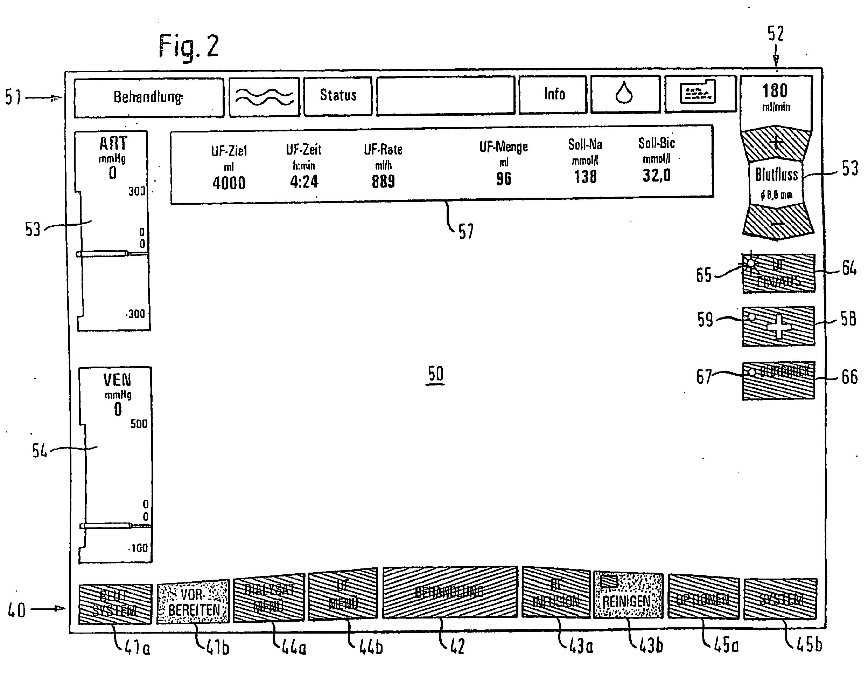

Fig. 2 zeigt eines erste Ansicht des Touchscreens 33 des Hämodialysegerätes. Am unteren Rand des Touchscreens sind verschiedene Modusmittel 40 in einer nebeneinanderliegenden Reihe dargestellt. Die Modusmittel 40 umfassen verschiedene Arten von Modusmitteln. Zunächst gibt es Blutbehandlungsvorbereitungsmittel 41a und 41b, Blutbehandlungsmittel 42 und Blutbehandlungsnachbereitungsmittel 43a und 43b, d.h. diese Modusmittel betreffen zeitliche Modi vor einer Blutbehandlung - hier einer Hämodialysebehandlung - , die eigentliche Blutbehandlung und nach einer Blutbehandlung. Des Weiteren sind Ergänzungsmodusmittel 44a, 44b und 45a und 45b vorgesehen, die die Eingabe ergänzender Informationen zu mehreren Zeitpunkten ermöglichen.

Auf dem Touchscreen 33 ist über den Modusmitteln 40 ein Bereich 50 angeordnet, auf dem je nach Betriebsmodus unterschiedliche Ansichten zu sehen sind. In den Randbereichen 51, 52 sind weitere Eingabe- und/oder Ausgabemittel (zum Beispiel Mittel 53 für die Blutpumpe 6) vorgesehen, die bestimmte Dateneingaben und die Anzeige erwünschter Informationen ermöglichen. Diese Randbereiche können unabhängig vom Betriebsmodus einen gleichartigen Aufbau haben oder davon abhängig sein.

Die in Fig. 2 gezeigte Ansicht zeigt das Erscheinigungsbild im Behandlung-Modus, das heißt während der eigentlichen Blutbehandlung. Im Behandlung-Modus wird auf der Anzeigefläche 50 die Datenleiste 57 angezeigt. Datenleiste 57 gibt Behandlungsfortschrittparameter wie die verbleibende Behandlungszeit und die bereits entzogene Ultrafiltrationsmenge wieder. Außerdem werden Eckwerte der Dialysierflüssigkeitszusammensetzung wie die Natrium- und die Bicarbonatkonzentration angezeigt. In den Bereichen 53 und 54 werden die durch die Drucksensoren 10 und 11 erfassten Werte angezeigt.

In der Datenleiste 52, die unter Anderem das Bedien- und Anzeigemittel 53 für die Blutpumpe 6 beinhaltet, befindet sich auch ein Notfallaktivierungsmittel 58. Das Notfallaktivierungsmittel 58 ist mit einem Symbol wie zum Beispiel einem Erste-Hilfe-Kreuz versehen, um die Bedeutung dieses Mittels dem Bedienpersonal unmittelbar zu vermitteln. Der Gesamtbereich des Notfallaktivierungsmittels 58 weist einen Teilbereich 59 auf, der in zwei unterschiedlichen Erscheinungsbildern wie zum Beispiel dunkel oder hell unterlegt dargestellt werden kann. Eine helle Darstellungsform belegt, dass das Notfallaktivierungsmittel 58 vorher betätigt wurde und die eingeleiteten Maßnahmen und Steuerungsprogramme weiterhin aktiv sind.

Unterhalb des Notfallaktivierungsmittels 58 ist ein Blutdruckmessungsmittel 66 dargestellt, das ebenfalls einen Teilbereich 67 ausweist, der in zwei unterschiedlichen Erscheinungsbildern wie zum Beispiel dunkel oder hell unterlegt dargestellt werden kann. Eine helle Unterlegung zeigt an, dass eine Blutdruckmessung im Gange ist.

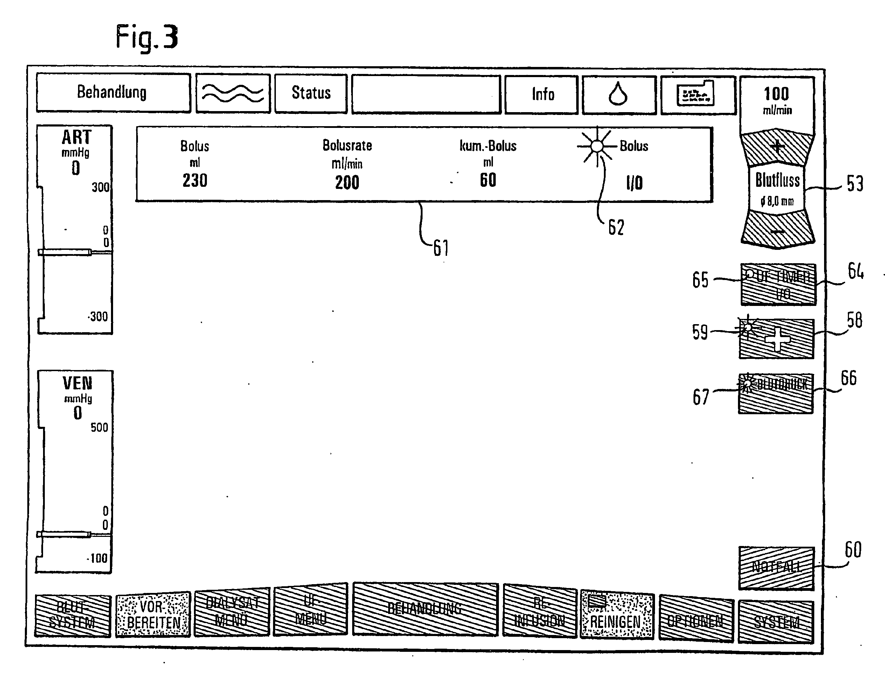

Durch Betätigung des Notfallaktivierungsmittels 58 sendet die Ein- und Ausgabeeinheit 32 ein Signal an die Steuer- und Auswerteeinheit 30. Nach Eingang des Signals fordert die Steuer- und Auswerteeinheit 30 von der Blutdruckmesseinheit 13 einen Blutdruckmesswert an, und weist die Dialysierflüssigkeitszubereitungseinheit 24, die Förder- und Bilanziereinrichtungen 22 und 23 sowie die Substituatpumpe 12 als Zugabeeinheit an, eine vorbestimmte Menge an Dialysierflüssigkeit mit einer vorbestimmten Rate in das Blut abzugeben. Gleichzeitig wird die Ein- und Ausgabeeinheit 30 angeweisen, die Einleitung dieser Maßnahmen durch ein sich von dem während der Behandlung angezeigten Erscheinungsbild abweichendes Erscheinungsbild eine Statusinformation über die Zugabe der Dialysierflüssigkeit in das Blut und die Blutdruckmessung anzuzeigen. Aus diesem Grund zeigt sich nach Betätigung des Notfallaktivierungsmittels 58 die in Fig. 3 gezeigte zweite Ansicht auf dem Touchscreen 33.

Innerhalb des Notfallaktivierungsmittels 58 ist eine Notfallprogrammanzeige 59 in Form einer simulierten grünen lichtemittierenden Diode (LED) vorgesehen, die nach der Aktivierung des Notfallprogramms entsprechend leuchtet. Dies gilt auch für den Teilbereich 67 in dem Blutdruckmessungsmittel 66, wobei der leuchtende Teilbereich 67 den aktiven Blutdruckmessvorgang symbolisiert. Gleichzeitig wird ein Notfallmenumittel 60 und ein vorher nicht sichtbares Ausgabefenster 61 eingeblendet. Mit Hilfe des Notfallmenumittels 60 kann jederzeit zu der in Fig. 3 gezeigten Ansicht gewechselt werden, solange das Notfallprogramm aktiv ist. Andernfalls wird das Notfallmenumittel 60 wieder ausgeblendet.

Das Blutbehandlungsmittel 42 wird nach Betätigung des Notfallaktivierungsmittels 58 in einer anderen Darstellungsform (in den Zeichnungen durch die Schraffur angedeutet) ähnlich der benachbarten Modusmittel 43a und 44b dargestellt, um zusätzlich zu symbolisieren, dass nun das Notfallprogramm aktiv ist. Stattdessen wird das Notfallmenumittel 60 in der Darstellungsform dargestellt, die der des Blutbehandlungsmittels 42 in Fig. 2 dargestellt war.

Das Ausgabefenster 61 gibt dem Anwender den Status über die Zugabe eines Flüssigkeitsbolus in das Blut des Patienten wieder. Die Steuer- und Auswerteeinheit 30 weist hierzu die Substituatpumpe 12 an, eine entsprechende Menge an steriler Dialysierflüssigkeit in die Blutrückführleitung 7 einzuspeisen. In dem Anzeigefenster 61 ist die Volumenmenge des Bolus, die gegenwärtige Förderrate der Pumpe 12, die bereits erfolgte Infusionsmenge des Bolus (kumulierter Bolus) sowie eine Bolusprogrammanzeige 62 (Bolus I/O) wiederum in Form einer simulierten LED zu erkennen. Während des Ablaufes des Programms zur Zugabe der Dialysierflüssigkeit, die im besonderen Kochsalz enthält, leuchtet die simulierte LED 62. Durch Berühren der Anzeige 62 kann die Substanzzugabe jederzeit unterbrochen, weitergeführt oder wiederholt werden. Auch kann die Bolusmenge und -rateneinstellung bei Bedarf durch Berühren der jeweiligen Zahlenangaben mit neuen Werten versehen werden.

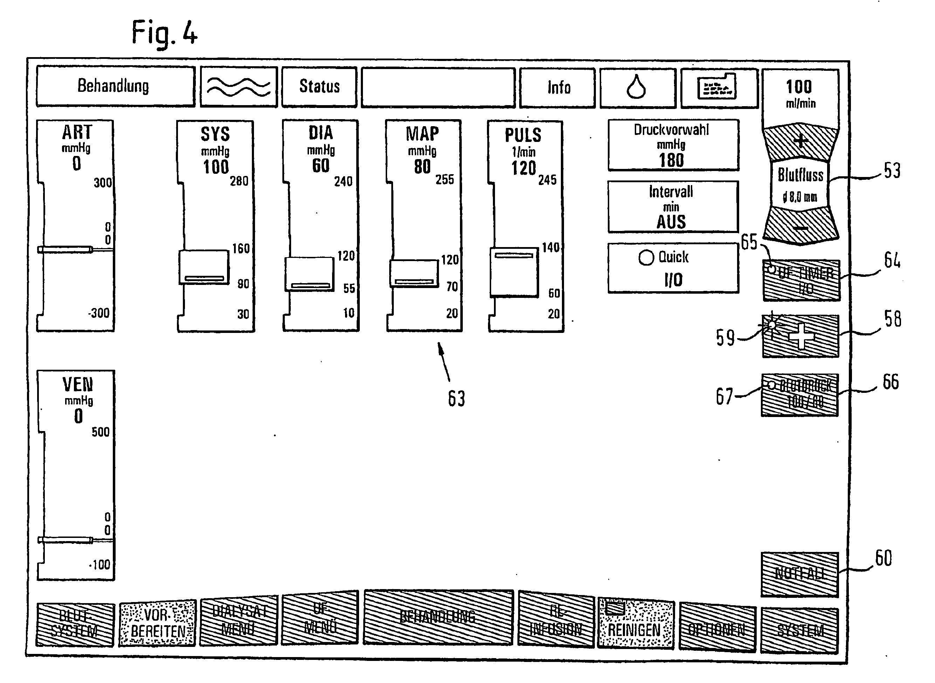

Gleichzeitig zu der Ansteuerung der Zugabeeinheit 12 sendet die Steuer- und Auswerteeinheit 30 ein Signal an die Blutdruckmesseinheit 13 zum Messen des Blutdrucks des Patienten. Die Steuereinheit 14 der Blutdruckmesseinheit 13 leitet darauf hin die Inflation der Manschette 16 ein, um eine automatisierte Blutdruckmessung am Patienten durchzuführen. Nach Abschluss dieser Messung übermittelt die Steuereinheit 14 die Daten für den systolischen (SYS), diastolischen (DIA) und den mittleren arteriellen Blutdruck (MAP) sowie den Herzpuls an die Steuer- und Auswerteeinheit 30. Diese sendet die Daten an die Ein- und Ausgabeeinheit 32, damit auf dem Touchscreen 33 in einem wiederum veränderten Erscheinungsbild, dass aufgrund dem zeitverzögerten Abschluss der Blutdruckmessung entsprechend zeitverzögert erscheint, Informationen über den Status der Blutdruckmessung zur Anzeige bringt (Fig. 4).

Bei der in Fig. 4 gezeigten Ansicht wird das Ausgabefenster 61 durch ein Ausgabefenster 63 ersetzt, auf dem die Werte für den systolischen (SYS), diastolischen (DIA) und den mittleren arteriellen Blutdruck (MAP) sowie den Herzpuls als Zahlenwert sowie in simulierter analoger Anzeige dargestellt werden. Gleichzeitig werden Mittel 64 eingeblendet, mit denen die Druckvorwahl des Inflationsdruckes für die Manschette, eine mögliche automatische Intervallmessung des Blutdruckes sowie über einen Ein- /Ausschalter eine sofortige Neumessung des Blutdruckes ermöglicht werden können. In dem Blutdruckmessungsmittel 66 wird der Teilbereich 67 wieder dunkel dargestellt, da der eigentliche Messvorgang abgeschlossen ist. Gleichzeitig werden die Messwerte für den systolischen und den diastolischen Blutdruck zusätzlich auf dem Blutdruckmessungsmittel 66 angezeigt. Diese Darstellung bleibt erhalten, wenn anstelle dem Ausgabefenster 63 andere Informationen darstellt werden.

Eine erneute Blutdruckmessung kann auch jederzeit ohne Einleitung der kompletten Notfallmaßnahmen durch Betätigung des Blutdruckmessungsmittels 66 veranlasst werden. Es kann auch vorgesehen sein, dass die in den Fig. 3 und 4 gezeigten Erscheinungsbilder kombiniert werden, das heißt insbesondere die Anzeigefenster 61 und 63 in einer gemeinsamen Ansicht dargestellt werden.

Gleichzeitig mit der Ansteuerung der Zugabeeinheit 12 sowie der Blutdruckmesseinheit 13 durch die Steuer- und Auswerteeinheit 30 nach Empfang des Signales von dem Notfallaktivierungsmittel 58 weist die Steuer- und Auswerteeinheit 30 auch die Blutpumpe 6 an, die Blutförderrate auf einen voreingestellten Wert zu reduzieren. Dieser Wert wird automatisch an dem Mittel 53 zur Bedienung der Blutpumpe 6 angezeigt (hier 100 ml/min). Zusätzlich wird die Ultrafiltrationseinheit 22, 23 angewiesen, die Ultrafiltration zu unterbrechen. Dies wird durch ein Ultrafiltrationaktivanzeigemittel 65 wiederum in Form einer simulierten LED angezeigt, die sich an einem Ein-/Ausschaltemittel 64 für die Ultrafiltrationseinheit 22, 23 befindet. Nach dem Drücken des Notfallsaktivierungsmittels 58 erlischt die simulierte LED 65.

Das Notfallprogramm, das nach der Betätigung des Notfallaktivierungsmittels 58 von der Steuer- und Auswerteeinheit 30 abgearbeitet wird, kann jederzeit durch nochmalige Betätigung des Notfallaktivierungsmittels 58 wieder deaktiviert werden. Die Bildschirmansicht kehrt dann zu der in Fig. 2 gezeigten Ansicht im Behandlungsmodus zurück, und die Behandlung wird mit den vorher eingestellten Einstellungen fortgeführt. Die Notfallprogrammanzeige 59 erlischt und das Notfallmenumittel 60 wird nicht mehr dargestellt.

Es kann auch vorgesehen sein, dass das Notfallprogramm nach einer eingestellten Zeit wieder automatisch in den Behandlungsmodus zurückkehrt, indem die Ultrafiltrationseinheit 22, 23 und/oder die Blutpumpe 6 auf die vorherigen Förderwerte eingestellt werden. Gleiches kann sich ergeben, wenn während des Notfallprogramms das Ein-/Ausschaltemittel 64 der Ultrafiltrationseinheit 22, 23 betätigt wird.

Es kann auch vorgesehen sein, dass die Steuer- und Auswerteeinheit 30 nach einer voreingestellten Zeit, in der das Notfallprogramm aktiv ist, ein Alarmsignal abgibt.

Über ein Anwender-Konfigurationsmenu (nicht gezeigt) kann vorgesehen werden, dass der Anwender einzelne Maßnahmen, die die Steuer- und Auswerteeinheit 30 nach Betätigung des Notfallaktivierungsmittels 58 veranlasst, aktiviert oder deaktiviert beziehungsweise Parameterwerte einstellt. Insbesondere hat es sich als vorteilhaft erwiesen, wenn die Deaktivierung der Ultrafiltrationseinheit sowie die Reduzierung der Blutförderrate, die Höhe der reduzierten Blutförderrate sowie die einzelnen Infusionswerte für die Substanzzugabe wie Flüssigkeitsvolumen und Förderrate eingestellt werden können.

Die Erfindung ermöglicht es dem Bedienpersonal, mit einer einzigen Bedienfunktion wesentliche diagnostische und therapeutische Maßnahmen während einer Hämodialysebehandlung im Falle einer hypotensiven Episode einzuleiten und gleichzeitig die Einleitung dieser Maßnahmen von dem Hämodialysegerät übersichtlich bestätigt zu bekommen. Dies beschleunigt die Umsetzung der einzuleitenden Maßnahmen und vermindert zur selben Zeit Fehlbedienungen, die es bei sich andeutenden Kreislaufproblemen von Patienten erst recht zu vermeiden gilt.

## Patentansprüche

1. Hämodialysegerät mit einem extrakorporalen Kreislauf, in dem Blut von einem Patienten zu der Blutkammer (3) eines durch eine semipermeable Membran (2) in zwei Kammern geteilten Hämodialysators (1) über eine Blutzuführleitung (5) und von der Blutkammer (3) zu dem Patienten über eine Blutrückführleitung (7) geführt wird und mit einer Blutpumpe (6) zur Förderung des Blutes durch den extrakorporalen Blutkreislauf,
mit einer Zugabeeinheit (12, 24) zur Zugabe einer Substanz in das Blut des Patienten,
mit einer Ultrafiltrationseinheit (22, 23) zum Entziehen von Flüssigkeit aus der zweiten Kammer (4) des Dialysators,
mit einer Blutdruckmesseinheit (13) zum Messen des Blutdrucks des Patienten,
mit einer Steuer- und Auswerteeinheit (30), die mit der Blutpumpe (6), der Zugabeeinheit (12, 24), der Ultrafiltrationseinheit (22, 23) und der Blutdruckmess-einheit (13) verbunden ist,
einer Ausgabeeinheit (32, 33) zur Ausgabe von Information, die mit der Steuer- und Auswerteeinheit (30) verbunden ist,
mit einer Eingabeeinheit (32) zur Eingabe von Daten und Anweisungen, die mit der Steuer- und Auswerteeinheit (30) verbunden ist,
wobei die Eingabeeinheit (32) ein Notfallaktivierungsmittel (58) umfasst, das bei Betätigung ein Signal an die Steuer- und Auswerteeinheit (30) abgibt, worauf diese von der Blutdruckmesseinheit (13) einen Blutdruckmesswert anfordern, die Zugabeeinheit (12, 24) anweist, in vorbestimmter Art die Substanz in das Blut abzugeben sowie die Ausgabeeinheit (32, 33) anweist, in einem sich von dem während der Behandlung gezeigten Erscheinungsbild abweichenden Erscheinungsbild (61, 63) eine Statusinformation über die Zugabe der Substanz in das Blut und die Blutdruckmessung anzuzeigen.

2. Hämodialysegerät nach Anspruch 1, wobei an die zweite Kammer (4) des Hämodialysators (1) eine von einer Dialysierflüssigkeitszubereitungseinheit (24) führende Dialysierflüssigkeitszuführleitung (20) zur Zuführung von Dialysierflüssigkeit angeschlossen ist,

3. Hämodialysegerät nach Anspruch 2, wobei die Dialysierflüssigkeitszubereitungseinheit (24) mit der Steuer- und Auswerteeinheit (30) verbunden ist.

4. Hämodialysegerät nach Anspruch 3, wobei die Dialysierflüssigkeitzubereitungseinheit (24) ein Teil der Zugabeeinheit ist.

5. Hämodialysegerät nach Anspruch 4, wobei die Substanz über die semipermeable Membran (2) des Hämodialysators (1) dem Blut zugeführt wird.

6. Hämodialysegerät nach Anspruch 4, wobei die Substanz über eine Substituatleitung (26), die mit der Dialysierflüssigkeitszubereitungseinheit (24) und der Blutzuführleitung (5) oder der Blutrückführleitung (7) in Verbindung steht, zugeführt wird.

7. Hämodialysegerät nach Anspruch 1, wobei der die Information ausgebende Teil der Ausgabeeinheit als Bildschirm (33) ausgeführt ist.

8. Hämodialysegerät nach Anspruch 7, wobei der Bildschirm (33) als beruhrungsempfindlicher Bildschirm ausgebildet ist.

9. Hämodialysegerät nach Anspruch 8, wobei das Notfallaktivieiungsmittel (58) als ein mit einem charakteristischen Symbol bezeichneter Bereich auf dem Bildschirm ausgebildet ist, dessen Berührung zur Aussendung des Signals führt.

10. Hämodialysegerät nach Anspruch 7, wobei die Steuer- und Auswerteeinheit (30) nach Empfang des Signals die Ausgabeeinheit (32) veranlasst, ein vorher nicht sichtbares Ausgabefenster (61, 63) zur gleichzeitigen oder nachfolgenden Anzeige der Statusinformation zur Blutdruckmessung und Substanzzugabe auf dem Bildschirm (33) sichtbar werden zu lassen.

11. Hämodialysegerät nach Anspruch 1, wobei die Steuer- und Auswerteeinheit (30) nach Empfang des Signals die Ultrafiltrationseinheit (22, 23) anweist, die Ultrafiltration auf einen vorgegebenen reduzierten Ultrafiltrationsratenwert zu reduzieren.

12. Hämodialysegerät nach Anspruch 11, wobei der reduzierte Ultrafiltrationsratenwert null ist.

13. Hämodialysegerät nach Anspruch 11, wobei die Möglichkeit der Steuer- und Auswerteeinheit (30), die Ultrafiltration bei Empfang des Signals zu reduzieren, Über die Eingabeeinheit (32) vom Benutzer aktiviert und dektiviert werden kann.

14. Hämodialysegerät nach Anspruch 13, wobei der reduzierte Ultrafiltrationsratenwert über die Eingabeeinheit (32) vom Benutzer vorgegeben werden kann.

15. Hämodialysegerät nach Anspruch 11, wobei die Steuer- und Auswerteeinheit (30) bei Empfang des Signals die Ausgabeeinheit (32) anweist, eine Statusinformation über die Reduzierung der Ultrafiltration anzuzeigen.

16. Hämodialysegerät nach Anspruch 15, wobei die Steuer- und Auswerteinheit (30) nach Empfang des Signals die Ausgabeeinheit (32) veranlasst, in einem vorher nicht sichtbaren Ausgabefenster die Statusinformation über die Reduzierung der Ultrafiltration auf dem Bildschirm (33) sichtbar werden zu lassen.

17. Hämofiltrationsgerät nach Anspruch 1, wobei die Steuer- und Auswerteeinheit (30) nach Empfang des Signals die Blutpumpe (6) anweist, die Blutförderrate auf einen reduzierten Blutförderratenwert zu reduzieren.

18. Hämodialysegerät nach Anspruch 17, wobei die Möglichkeit der Steuer- und Auswerteeinheit (30), die Blutförderrate bei Empfang des Signals zu reduzieren, über die Eingabeeinrichtung (32) vom Benutzer aktiviert und dektiviert werden kann.

19. Hämodialysegerät nach Anspruch 18, wobei der reduzierte Blutförderratenwert über die Eingabeeinheit (32) vom Benutzer vorgegeben werden kann.

20. Hämodialysegerät nach Anspruch 17, wobei die Steuer- und Auswerteeinheit (30) bei Empfang des Signals die Ausgabeeinheit (32) anweist, eine Statusinformation über die Reduzierung der Blutforderrate anzuzeigen.

21. Hämodialysegerät nach Anspruch 20, wobei die Steuer- und Auswerteinheit (30) nach Empfang des Signals die Ausgabeeinheit (32) veranlasst, in einem vorher nicht sichtbaren Ausgabefenster die Statusinformation über die Reduzierung der Blutförderrate auf dem Bildschirm (33) sichtbar werden zu lassen.

## Claims

1. A hemodialysis machine having an extracorporeal circulation, in which blood from a patient is carried through a blood supply line (5) to the blood chamber (3) of a hemodialyzer (1), which is divided by a semipermeable membrane (2) into two chambers, and is carried from the blood chamber (3) to the patient through a blood return line (7), and having a blood pump (6) for conveying the blood through the extracorporeal blood circulation,
having a dosing unit (12, 24) for adding a substance to the patient's blood,
having an ultrafiltration unit (22, 23) for removing fluid from the second chamber (4) of the dialyzer,
having a blood pressure measurement unit (13) for measuring the patient's blood pressure,
having a control and evaluation unit (30), which is connected to the blood pump (6), the dosing unit (12, 24), the ultrafiltration unit (22, 23) and the blood pressure measurement unit (13),
having an output unit (32, 33) for output of information, which is connected to the control and evaluation unit (30),
having an input unit (32) for input of data and instructions, which is connected to the control and evaluation unit (30),
wherein the input unit (32) comprises an emergency activation means (58), which delivers a signal to the control and evaluation unit (30) when activated, whereupon it requests a measured blood pressure value from the blood pressure measuring unit (13), instructs the dosing unit (12, 24) to dispense the substance to the blood in a predetermined manner and instructs the output unit (32, 33), to display a status information about the addition of the substance to the blood and the blood pressure measurement in an appearance (61, 63) differing from that displayed during the treatment.

2. The hemodialysis machine according to Claim 1, wherein a dialysis fluid supply line (20) for supplying dialysis fluid leading from a dialysis fluid preparation unit (24) is connected to the second chamber (4) of the hemodialyzer (1).

3. The hemodialysis machine according to Claim 2, wherein the dialysis fluid preparation unit (24) is connected to the control and evaluation unit (30).

4. The hemodialysis machine according to Claim 3, wherein the dialysis fluid preparation unit (24) is part of the dosing unit.

5. The hemodialysis machine according to Claim 4, wherein the substance is added to the blood via the semipermeable membrane (2) of the hemodialyzer (1).

6. The hemodialysis machine according to Claim 4, wherein the substance is supplied via a substituate line (26), which is connected to the dialysis fluid preparation unit (24) and the blood supply line (5) or the blood return line (7).

7. The hemodialysis machine according to Claim 1, wherein the part of the output unit which outputs the information is designed as a display screen (33).

8. The hemodialysis machine according to Claim 7, wherein the display screen (3) is designed as a touch-sensitive screen.

9. The hemodialysis machine according to Claim 8, wherein the emergency activation means (58) is designed as an area on the display screen identified with a characteristic symbol, so that touching this area leads to transmission of the signal.

10. The hemodialysis machine according to Claim 7, wherein the control and evaluation unit (30) prompts the output unit (32) after receiving the signal to make a previously invisible output window (61, 63) visible on the display screen (33) for simultaneous or subsequent display of the status information for the blood pressure measurement and addition of the substance.

11. The hemodialysis machine according to Claim 1, wherein the control and evaluation unit (30) instructs the ultrafiltration unit (22, 23) after receiving the signal to reduce the ultrafiltration to a predetermined reduced ultrafiltration rate.

12. The hemodialysis machine according to Claim 11, wherein the reduced ultrafiltration rate is zero.

13. The hemodialysis machine according to Claim 11, wherein the possibility of the control and evaluation unit (30) reducing ultrafiltration on reception of the signal can be activated and deactivated by the user via the input unit (32).

14. The hemodialysis machine according to Claim 13, wherein the reduced ultrafiltration rate can be preselected by the user via the input unit (32).

15. The hemodialysis machine according to Claim 11, wherein the control and evaluation unit (30) instructs the output unit (32) on receiving the signal to display status information about the reduction of the ultrafiltration.

16. The hemodialysis machine according to Claim 15, wherein the control and evaluation unit (30) prompts the output unit (32) on reception of the signal to make the status information about the reduction in ultrafiltration visible on the display screen (3) in an output window which was not previously visible.

17. The hemofiltration machine according to Claim 1, wherein the control and evaluation unit (30), after receiving the signal, instructs the blood pump (6) to reduce the blood delivery rate to a reduced blood delivery rate.

18. The hemodialysis machine according to Claim 17, wherein the possibility of the control and evaluation unit (30) to reduce the blood delivery rate on receiving the signal can be activated and deactivated by the user via the input device (32).

19. The hemodialysis machine according to Claim 18, wherein the reduced blood delivery rate can be preselected by the user via the input unit (32).

20. The hemodialysis machine according to Claim 17, wherein the control and evaluation unit (30) instructs the output unit (32) on receiving the signal to display status information about the reduction in the blood delivery rate.

21. The hemodialysis machine according to Claim 20, wherein the control and evaluation unit (30), after receiving the signal, prompts the output unit (32) to make the status information about the reduction in the blood delivery rate visible on the display screen (33) in an output window which was not previously visible.

## Revendications

1. Appareil d'hémodialyse à circuit extracorporel, dans lequel du sang d'un patient est conduit vers la cellule sanguine (3) d'un hémodialyseur (1) divisé en deux chambres par une membrane semi-perméable (2) par une conduite d'afflux sanguin (5) et de la cellule sanguine (3) vers le patient par une conduite de reflux sanguin (7) et comportant une pompe sanguine (6) pour convoyer le sang dans le circuit sanguin extracorporel,
comportant une unité d'apport (12, 24) permettant d'apporter une substance dans le sang du patient,
une unité d'infiltration (22, 23) permettant de retirer du liquide de la seconde chambre (4) du dialyseur,
une unité de mesure de tension artérielle (13) pour mesurer la tension artérielle du patient,
une unité de commande et d'exploitation (30) qui est reliée à la pompe sanguine (6), à l'unité d'apport (12, 24), à l'unité de filtration (22, 23) et à l'unité de mesure de tension artérielle (13),
une unité d'édition (32, 33) servant à l'édition d'information et qui est reliée à l'unité de commande et d'exploitation (30),
une unité de saisie (32) pour la saisie de données et d'instructions et qui est reliée à l'unité de commande et d'exploitation (30),
l'unité de saisie (32) comprenant un moyen d'activation d'urgence qui émet en cas d'actionnement un signal vers l'unité de commande et d'exploitation (30), sur quoi celle-ci demande à l'unité de mesure de tension artérielle (13) une valeur de mesure de tension artérielle, ordonne à l'unité d'apport (12, 24) de diffuser la substance dans le sang de manière prédéfinie, ordonne à l'unité d'édition (32, 33) d'afficher, dans une image de représentation (61, 63) différente de l'image de représentation montrée pendant le traitement, une information d'état sur l'apport de la substance dans le sang et sur la mesure de tension artérielle.

2. Appareil d'hémodialyse selon la revendication 1, une conduite d'acheminement de liquide de dialyse (20) partant d'une unité de préparation de liquide de dialyse (24) étant raccordée à la seconde cellule (4) du dialyseur (1) pour l'apport de liquide de dialyse.

3. Appareil d'hémodialyse selon la revendication 2, l'unité de préparation de liquide de dialyse (24) étant raccordée à l'unité de commande et d'exploitation (30).

4. Appareil d'hémodialyse selon la revendication 3, l'unité de préparation de liquide de dialyse (24) faisant partie de l'unité d'apport.

5. Appareil d'hémodialyse selon la revendication 4, la substance étant apportée au sang par l'intermédiaire de la membrane semi-perméable (2) de l'hémodialyseur (1).

6. Appareil d'hémodialyse selon la revendication 4, la substance étant apportée par l'intermédiaire d'une conduite de produit de substitution (26) qui est en liaison avec l'unité de préparation de liquide de dialyse (24) et la conduite d'afflux sanguin (5) ou la conduite de reflux sanguin (7).

7. Appareil d'hémodialyse selon la revendication 1, l'élément émetteur d'information de l'unité d'édition se présentant sous forme d'un écran (33).

8. Appareil d'hémodialyse selon la revendication 7, l'écran (7) se présentant sous forme d'un écran sensible au toucher.

9. Appareil d'hémodialyse selon la revendication 8, le moyen d'activation d'urgence (58) se présentant sous la forme d'une zone identifiée par un symbole caractéristique sur l'écran et dont le toucher provoque l'émission du signal.

10. Appareil d'hémodialyse selon la revendication 7, l'unité de commande et d'exploitation (30) amenant, après réception du signal, l'unité d'édition (32) à rendre visible à l'écran (33) une fenêtre d'édition (61, 63) auparavant non visible ou à afficher simultanément ou successivement l'information d'état sur la mesure de tension artérielle et l'apport de substance.

11. Appareil d'hémodialyse selon la revendication 1, l'unité de commande et d'exploitation (30) ordonnant, après réception du signal, à l'unité d'ultrafiltration (22, 23) de réduire l'ultrafiltration à une valeur de vitesse d'ultrafiltration réduite prédéfinie.

12. Appareil d'hémodialyse selon la revendication 11, la valeur de vitesse d'ultrafiltration réduite étant de zéro.

13. Appareil d'hémodialyse selon la revendication 11, la possibilité offerte à l'unité de commande et d'exploitation (30) de réduire l'ultrafiltration à la réception du signal pouvant être activée et désactivée par l'utilisateur via l'unité de saisie (32).

14. Appareil d'hémodialyse selon la revendication 13, la valeur de taux d'ultrafiltration réduite pouvant être prédéterminée par l'utilisateur via l'unité de saisie (32).

15. Appareil d'hémodialyse selon la revendication 11, l'unité de commande et d'exploitation (30) ordonnant, après réception du signal, à l'unité d'édition (32) d'afficher une information d'état sur la réduction de l'ultrafiltration.

16. Appareil d'hémodialyse selon la revendication 15, l'unité de commande et d'exploitation (30) amenant, après réception du signal, l'unité d'édition (32) à rendre visible à l'écran (33) dans une fenêtre d'édition auparavant non visible l'information d'état sur la réduction de l'ultrafiltration.

17. Appareil d'hémodialyse selon la revendication 1, l'unité de commande et d'exploitation (30) ordonnant, après réception du signal, à la pompe sanguine (6) de réduire la vitesse de transport du sang à une vitesse de transport de sang réduite.

18. Appareil d'hémodialyse selon la revendication 17, la possibilité offerte à l'unité de commande et d'exploitation (30) de réduire la vitesse de transport du sang à réception du signal pouvant être activée et désactivée par l'utilisateur via l'unité de saisie (32).

19. Appareil d'hémodialyse selon la revendication 18, la vitesse de transport du sang réduite pouvant être prédéterminée par l'utilisateur via l'unité de saisie (32).

20. Appareil d'hémodialyse selon la revendication 17, l'unité de commande et d'exploitation (30) ordonnant, à réception du signal, à l'unité d'édition (32) d'afficher une information d'état sur la réduction de la vitesse de transport du sang.

21. Appareil d'hémodialyse selon la revendication 20, l'unité de commande et d'exploitation (30) amenant, après réception du signal, l'unité d'édition (32) à afficher à l'écran (33) dans une fenêtre d'information auparavant non visible l'information d'état sur la réduction de la vitesse de transport du sang.
